# EUROPÄISCHE PATENTSCHRIFT

(11) **EP 2 327 356 B1**
(45) Veröffentlichungstag und Bekanntmachung des Hinweises auf die Patenterteilung: **19.03.2014**
(21) Anmeldenummer: 10014337.9
(22) Anmeldetag: 05.11.2010
(51) Int. Cl.: A61B 1/04, A61B 1/06, A61B 1/00, A61B 1/015, A45C 9/00

(54) **Medizinische Vorrichtung zur Unterstützung einer endoskopischen Untersuchung**
Medical support device for endoscopy
Dispositif médical pour soutenir un examen endoscopique

(30) Priorität: 30.11.2009 DE 102009056108
(43) Veröffentlichungstag der Anmeldung: 01.06.2011
(73) Patentinhaber: Karl Storz GmbH & Co. KG, 78532 Tuttlingen (DE)
(72) Erfinder: Geisser, Romana, 78176 Blumberg (DE); Hensler, Fritz, 78579 Neuhausen (DE); Rebholz, Clemens, 88690 Uhldingen-Mühlhofen (DE); Schwarz, Peter, 78532 Tuttlingen-Nendingen (DE)

(56) Entgegenhaltungen:
- EP-A1- 0 437 229
- EP-A1- 1 977 693
- EP-A1- 2 092 892
- WO-A1-97/15144
- WO-A1-2008/117304
- WO-A2-2004/041096
- WO-A2-2007/115225
- CN-Y- 2 549 897
- DE-U1- 20 119 393
- US-A- 4 012 686
- US-A- 5 885 214
- US-A1- 2004 165 393
- US-A1- 2006 287 575
- US-A1- 2007 224 571
- US-A1- 2008 175 020
- US-B1- 6 257 741

## Beschreibung

Die vorliegende Beschreibung bezieht sich auf eine medizinische Vorrichtung zur Unterstützung einer endoskopischen Untersuchung mit einer Lichtquelle zum Erzeugen von Licht zur Beleuchtung eines mit einem Endoskop zu betrachtenden Gegenstands und einem Bildschirm zum Betrachten eines mittels des Endoskops erfassten Bilds.

Endoskopische medizinische Untersuchungen und Eingriffe wurden lang fast ausschlieβ-lich in Operationssälen oder anderen medizinischen Behandlungsräumen durchgeführt. Die für die endoskopischen Untersuchungen erforderlichen Lichtquellen standen beispielsweise in feststehenden oder auf Rollen beweglichen Racks bzw. Regalen. Mit Verwendung einer Kamera am proximalen Ende des Endoskops und später mit der Einführung des Videoendoskops mit einer Kamera am distalen Ende des Endoskops kam ein Bildschirm zur Darstellung der von der Kamera erfasste Bilder hinzu, der an einem geeigneten Ort aufgestellt oder aufgehängt wurde. Zur Dokumentation wurden die von der Kamera erfassten Bilder auch einer Speichereinheit (Videorecorder, DVD-Brenner, Festplatten-laufwerk oder Ähnliches) zugeführt, das als weiteres separates Gerät im Behandlungsraum aufgestellt wurde.

In zunehmendem Maße wurden endoskopische Untersuchungen und Eingriffe medizinischer oder auch nicht-medizinischer Natur außerhalb dafür eingerichteter und ausgerüsteter Behandlungsräume durchgeführt. Beispiele sind die Endoskopie in der Notfallmedizin, in umfassen und so in Kombination mit einem Videoendoskop oder einem Endoskop mit Kamerakopf am proximalen Ende endoskopische Untersuchen ermöglichen.

Aus der CN 2549897 Y ist ein Gerät zur Verbindung mit einem Endoskop bekannt, mit einer Lichtquelle und einem Bildschirm, der abgesetzt und senkrecht auf der Oberseite des Gehäuses des Gerätes angebracht ist.

Aus der US 5,885,214 A ist ein Gerät für die Otologie bekannt mit einem Gehäuse und einem an dessen Frontfläche angebrachten Bildschirm. Dieses erweist sich als unhandlich und wenig transportfreundlich.

Diese herkömmlichen integrierten Geräte weisen jedoch eine Reihe von Nachteilen auf. Insbesondere lassen sie es, mit klappbaren Bildschirmen oder Standfüßen ausgerüstet, an der wünschenswerten Robustheit und Standfestigkeit mangeln.

Eine Aufgabe der vorliegenden Erfindung besteht darin, eine verbesserte medizinische Vorrichtung zur Unterstützung einer endoskopischen Untersuchung zu schaffen, das sich insbesondere durch gute Standfestigkeit, gute Robustheit, gute Ablesbarkeit und Transportierbarkeit auszeichnet.

Diese Aufgabe wird durch die Gegenstände des unabhängigen Anspruchs gelöst.

Weiterbildungen sind in den abhängigen Ansprüchen angegeben.

Ausführungsformen der vorliegenden Erfindung beruhen auf der Idee, bei einer medizinischen Vorrichtung zur Unterstützung einer endoskopischen Untersuchung die Frontfläche und die Rückseitenfläche gegenüber der Bodenfläche zur Rückseite hin zu neigen, und zwar um einen Winkel zwischen 5 Grad und 15 Grad, insbesondere um einen Winkel zwischen 7 Grad und 10 Grad. Bei einem Ausführungsbeispiel hat sich ein Winkel zwischen 8 Grad und 9 Grad besonders bewährt. Die Neigung der Frontfläche mit einem integrierten Bildschirm verbessert bei vielen praktischen Anwendungen, bei denen die medizinische Vorrichtung beispielsweise auf einem Tisch oder auf dem Boden eines Stalls steht, die Ablesbarkeit des Bildschirms, Gleichzeitig kann bei der genannten Neigung der Frontfläche und der Rückseitenfläche gegenüber der Bodenfläche noch eine gute Standfestigkeit der medizinischen Vorrichtung erzielt werden.

Herkömmlich wird zur Erzielung einer Standfestigkeit beispielsweise ein Gehäuse in Form eines liegenden Quaders ausgebildet, bei dem eine der beiden größten rechteckigen Außenflächen die Bodenfläche bzw. Standfläche bildet. Alternativ sind beispielsweise ausklappbare Standfüße oder Leisten zur Verbreiterung der Basis vorgesehen. Die vorliegende Erfindung beruht auf der Erkenntnis, dass auch ein Gehäuse, das näherungsweise die Form eines stehenden Quaders aufweist, eine hohe Standfestigkeit aufweisen kann, auch bei einer leichten Neigung der Frontfläche und der Rückseitenfläche nach hinten. Durch die leichte Neigung nach hinten wird der Schwerpunkt des zur Gesamtmasse der medizinischen Vorrichtung erheblich beitragenden Bildschirms nach hinten und damit in Richtung zur Mitte der Bodenfläche verschoben. Durch eine bevorzugte Anordnung schwerer Komponenten bzw. Bestandteile der medizinischen Vorrichtung in der unteren Hälfte, noch besser im unteren Drittel des Gehäuses der medizinischen Vorrichtung, werden deren Schwerpunkte durch die Neigung des Gehäuses nicht wesentlich nach hinten verschoben.

Damit schafft die vorliegende Erfindung eine medizinische Vorrichtung zur Unterstützung einer endoskopischen Untersuchung, die auch ohne eine Schwenkbarkeit eines integrierten Bildschirms mittels eines Scharniers eine gute Ablesbarkeit des Bildschirms ermöglicht.

Gleichzeitig ermöglicht die an einen Quader angenäherte Form des Gehäuses eine Kompaktheit, die der Transportabilität zugute kommt, und die trotz oder ― angesichts des schweren Bildschirms an der Frontseite ― gerade wegen der leichten Neigung der Frontseite nach hinten ausgewogene Schwerpunktlage schafft eine gute Standfestigkeit der medizinischen Vorrichtung.

Eine medizinische Vorrichtung zur Unterstützung einer endoskopischen Untersuchung umfasst ein Gehäuse mit einer Bodenfläche, einer Frontfläche und einer Rückseitenfläche, eine Lichtquelle, die in dem Gehäuse angeordnet ist, zum Erzeugen von Licht zur Beleuchtung eines mit einem Endoskop zu betrachtenden Gegenstands. Des Weiteren umfasst eine medizinische Vorrichtung zur Unterstützung einer endoskopischen Untersuchung eine Kopplungseinrichtung zum unmittelbaren oder mittelbaren Koppeln eines proximalen Endes eines Endoskops an die medizinische Vorrichtung, und zum Übertragen von Licht von der medizinischen Vorrichtung zu dem Endoskop und einen Bildschirm, der an der Frontfläche des Gehäuses angeordnet ist, zum Betrachten eines mittels des Endoskops erfassten Bilds, wobei die Frontfläche und die Rückseitenfläche gegenüber der Bodenfläche jeweils um einen Winkel zwischen 5 Grad und 15 Grad zur Rückseite hin geneigt sind. Die Bodenfläche ist derjenige (beispielsweise von Standfüßen und nachfolgend beschriebenen optionalen Befestigungseinrichtungen abgesehen) im Wesentlichen ebene Bereich der äußeren Oberfläche des Gehäuses, der vorgesehen und ausgebildet ist, um bei der für die medizinische Vorrichtung vorgesehenen Verwendung im Wesentlichen parallel zu einer Tischplatte, einem Boden oder einer anderen Oberfläche, auf der die medizinische Vorrichtung steht, angeordnet ist. Die Frontfläche ist insbesondere ebenfalls im Wesentlichen eben, Abweichungen von einer vollständig ebenen Gestalt der Frontfläche ergeben sich beispielsweise durch Tastenknöpfe, andere Bedienelemente, elektrische Steckbuchsen oder eine etwas versenkte Anordnung des Bildschirms. Diese Abweichungen betragen in einem bevorzugten Ausführungsbeispiel jedoch typischerweise nur wenige Millimeter. Bei dem unten beschriebenen Ausführungsbeispiel stehen Tasten an der Frontfläche gegenüber einem dem Bildschirm zumindest an drei Seiten umschließenden ebenen Rand um weniger als 3 mm über, und die Frontfläche des Bildschirms ist gegenüber dem genannten Rand um weniger als 3 mm versenkt. Die Rückseitenfläche ist der größte, im Wesentlichen ebene zusammenhängende oder nicht zusammenhängende Abschnitt der Oberfläche des Gehäuses an dessen Rückseite. Außer diesem im Wesentlichen ebenen Abschnitt kann die Rückseite des Gehäuses insbesondere Vertiefungen und Ausnehmungen aufweisen. Die Rückseitenfläche kann geringfügige Abweichungen von der ebenen Gestalt aufweisen, beispielsweise in Form von erhabener oder vertiefter Schrift oder anderer Symbole, in Form von Lüftungsgittern oder Lüftungsöffnungen, bündig oder im Wesentlichen bündig eingesetzten Abdeckungen, Vertiefungen, in denen Schraubenköpfe angeordnet sind.

Der Winkel, um den die Frontfläche und die Rückseitenfläche gegenüber der Bodenfläche geneigt sind, bzw. die Abweichung des Winkels zwischen der Frontfläche und der Bodenfläche von 90 Grad liegt insbesondere im Bereich von 7 Grad bis 10 Grad oder im Bereich von 8 Grad bis 9 Grad. Wie bereits erwähnt, verbessert diese Neigung der Frontfläche und damit des Bildschirms an der Frontfläche die Ablesbarkeit des Bildschirms. Gleichzeitig wird der relativ hoch (näherungsweise in der Mitte des Bildschirms) liegende Schwerpunkt des Bildschirms durch die Neigung der Frontfläche nach hinten verschoben, wodurch die Standfestigkeit der medizinischen Vorrichtung verbessert werden kann.

Bei einer medizinischen Vorrichtung, wie sie oben beschrieben ist, ist die Kopplungseinrichtung zum Koppeln eines proximalen Endes eines Endoskops direkt oder indirekt über ein Lichtleitkabel an die medizinische Vorrichtung ausgebildet. Die Kopplungseinrichtung kann unter einem Winkel zwischen 50 Grad und 70 Grad zur Frontfläche angeordnet sein. Der Winkel zwischen 50 Grad und 70 Grad ist insbesondere der Winkel zwischen einer Richtung, in der eine Kupplung eines Lichtleitkabels oder eines proximalen Endes eines Endoskops in die Kopplungseinrichtung eingeführt werden kann, und der Flächennormale der Frontfläche.

Wenn dieser Winkel 0 Grad beträgt, erschwert die genau nach vorn bzw. parallel zur Flächennormale der Frontfläche vom Gehäuse abstehende Kupplung des Lichtleitkabels oder das entsprechend ausgerichtete proximale Ende des Endoskops die Bedienung von Schaltem und Tasten an der Frontfläche. Wenn der Winkel 90 Grad beträgt, die Kupplung eines Lichtleitkabels oder die Kupplung am proximalen Ende eines Endoskops also von der Seite her in die Kopplungseinrichtung am Gehäuse der medizinischen Vorrichtung einzuführen ist, steht die Kupplung bzw. das Lichtleitkabel bzw. das proximale Ende des Endoskops sehr weit seitlich ab. In beengten räumlichen Verhältnissen, die beispielsweise in der Notfallmedizin und in der Veterinärmedizin häufig auftreten, bedeutet dies nicht nur eine Beschränkung beim Aufstellen der medizinischen Vorrichtung, sondem auch ein erhöhtes Risiko einer Beschädigung.

Demgegenüber bietet ein Winkel zwischen 50 Grad und 70 Grad, insbesondere ein Winkel von 60 Grad einerseits einen reduzierten seitlichen Platzbedarf bzw. eine insgesamt kompaktere Anordnung und andererseits einen ungehinderten Zugriff auf alle Bedienelemente an der Frontfläche.

Bei einer medizinischen Vorrichtung, wie sie hier beschrieben ist, nimmt die Lichtquelle einen Raumbereich in dem Gehäuse ein, der ausschließlich in der an die Bodenfläche angrenzenden Hälfte des Gehäuses angeordnet ist, insbesondere ausschließlich in dem an die Bodenfläche angrenzenden Drittel des Gehäuses. Der von der Lichtquelle eingenom-mene Raumbereich grenzt insbesondere an die Bodenfläche an. Das Gleiche kann für die Einrichtungen zur Versorgung der medizinischen Vorrichtung mit elektrischer Leistung aus einem öffentlichen Netz, insbesondere für einen Transformator, und für eine Pumpe, einen Verdichter oder ein Gebläse zum Fördern eines Fluids gelten. Neben dem Bildschirm, dessen Schwerpunkt durch die Neigung der Frontfläche zur Rückseite hin verschoben ist, können die Lichtquelle und die Einrichtungen zur Leistungsversorgung die schwersten Bestandteile bzw. Komponenten der medizinischen Vorrichtung sein. Durch die beschriebene Anordnung der Lichtquelle, der Einrichtungen zur Leistungsversorgung und der Pumpe, des Verdichters oder Gebläses hat die Neigung des Gehäuses zur Rückseite hin― von der beschriebenen positiven Wirkung auf den Schwerpunkt des Bildschirms abgesehen― nur eine geringe Auswirkung auf die Lage des Schwerpunkts der medizinischen Vorrichtung. Die beschriebene Anordnung der Lichtquelle, der Einrichtungen zur Leistungsversorgung und der Pumpe, dem Verdichter oder Gebläse leistet deshalb einen besonderen Beitrag dazu, dass durch die Neigung der Frontfläche mit dem Bildschirm eine gute Ablesbarkeit des Bildschirms mit einer guten Standfestigkeit der medizinischen Vorrichtung kombinierbar ist.

Eine medizinische Vorrichtung, wie sie hier beschrieben ist, kann an der Bodenfläche eine Befestigungseinrichtung, insbesondere mehrere Befestigungseinrichtungen, zum Befestigen des Gehäuses der medizinischen Vorrichtung zumindest entweder an einem Transportkoffer für die medizinische Vorrichtung oder an einem Stativ oder an oder in einem Regal oder an einer anderen Vorrichtung aufweisen. Die Befestigungseinrichtung ist beispielsweise zur Befestigung der medizinischen Vorrichtung an einer Außenfläche und/oder an einer Innenfläche eines Transportkoffers ausgebildet. Beispielsweise wird die medizinische Vorrichtung so in einem Transportkoffer befestigt, dass sie unmittelbar nach einem Aufklappen des Transportkoffers, also ohne die medizinische Vorrichtung zuvor von dem Transportkoffer mechanisch zu trennen, zur Unterstützung einer endoskopischen Untersuchung verwendet werden kann. Alternativ oder zusätzlich kann die Befestigungsvorrichtung zur Befestigung der medizinische Vorrichtung an oder in einem Regal vorgesehen sein, beispielsweise einem Regal in einem medizinischen Behandlungsraum.

Die Befestigungseinrichtung an der Bodenfläche der medizinischen Vorrichtung ist insbesondere eine Nut mit Schwalbenschwanz-förmigem bzw. trapezförmigem Querschnitt oder mit T-förmigem Querschnitt. Der Querschnitt der Nut kann sich von einem Ende der Nut zum anderen hin reduzieren, um eine Klemmwirkung mit einem entsprechenden Gegenstück zu bewirken. Anstelle der oder zusätzlich zu der einen oder mehreren Nuten können eine oder mehrere Schraubgewinde, Bajonettverschlüsse oder andere Einrichtung zum formschlüssigen Befestigung vorgesehen sein. Anstelle einer Einrichtung zur formschlüssigen Befestigung oder zusätzlichen zu dieser können eine oder mehrere Einrichtungen an der Bodenfläche des Gehäuses vorgesehen sein, die bei einer kraftschlüssigen bzw. reibschlüssigen Befestigung der medizinischen Vorrichtung an einer Außenfläche eines Transportkoffers, eines Stativs oder einer anderen Vorrichtung die Normalkraft erhöhen. Dazu zählen ein Vakuumsaugfuß, eine Magnethalterung, Spannriemen, Spannbacken und Spannschlösser.

Die beschriebenen Befestigungseinrichtungen können, erforderlichenfalls mit den entsprechenden komplementären Einrichtungen an einem Transportkoffer, einem Stativ oder einer anderen Vorrichtung, eine zuverlässige aber leicht und schnell herstellbare und lösbare mechanische Befestigung der medizinischen Vorrichtung ermöglichen. Gerade die Befestigung der medizinischen Vorrichtung an einem Transportkoffer, der zum Transport der medizinischen Vorrichtung bei vielen Anwendungen ohnehin zum Einsatz kommt, ermöglicht eine weitere Erhöhung der Standfestigkeit der medizinischen Vorrichtung bei geringen zusätzlichen Kosten. Die medizinische Vorrichtung kann in Form eines Systems aus mehreren Komponenten neben dem Gehäuse mit der Lichtquelle, der Kopplungseinrichtung und dem Bildschirm einen Transportkoffer umfassen, der zur vollständigen Aufnahme der medizinischen Vorrichtung bei deren Transport ausgebildet ist und eine Befestigungseinrichtung an einer Außenfläche aufweist, die zu der Befestigungseinrichtung an der Bodenfläche des Gehäuses komplementär ist.

Es ist selbstverständlich auch möglich, dass die Befestigungseinrichtung an der Bodenfläche der medizinischen Vorrichtung insbesondere ein Nut mit Schwalbenschwanz-förmigem bzw. trapezförmigem Querschnitt oder mit T-förmigem Querschnitt ist. Die Befestigungseinrichtungen an einem Transportkoffer, einem Stativ oder einer anderen Vorrichtung sind entsprechend komplementär.

Bei einer medizinischen Vorrichtung, wie sie hier beschrieben ist, kann die Rückseitenfläche U-förmig eine Ausnehmung umschließen, in der eine Befestigungseinrichtung zur Befestigung eines Akkumulators an der medizinischen Vorrichtung angeordnet ist. Dieser Akkumulator kann zur Versorgung der medizinischen Vorrichtung mit dieser mechanisch und elektrisch gekoppelt werden, um die medizinische Vorrichtung an Orten betreiben zu können, an denen kein elektrisches Leistungsversorgungsnetz verfügbar ist. Die Ausnehmung und der Akkumulator sind hinsichtlich Form und Größe insbesondere so aufeinander abgestimmt, dass der Akkumulator nicht aus der Ausnehmung herausragt, wenn er an der Befestigungseinrichtung in der Ausnehmung befestigt ist. Dies bedeutet insbesondere, dass der an der Befestigungseinrichtung in der Ausnehmung befestigte Akkumulator nicht oder nicht wesentlich über die Ebene übersteht, in der die Rückseitenfläche liegt, bzw. die durch die Rückseitenfläche definiert ist.

Die Befestigungseinrichtung in der Ausnehmung kann auch zur alternativen Befestigung des genannten Akkumulators oder eines Kühlkörpers an der medizinischen Vorrichtung oder der medizinischen Vorrichtung an einem Träger, beispielsweise einem Wand- oder Deckenträger, einem Gerätewagen oder einem Regal, ausgebildet sein. Dazu entspricht die Befestigungseinrichtung insbesondere dem VESA-Standard (VESA = Video Electronic Standard Association) für Wand- und Deckenhalterung von Flachbildschirmen. Insbesondere umfasst die Befestigungseinrichtung vier Gewindelöcher an den Ecken eines Quadrats mit einer Seitenlänge von 100 mm, wobei jedes Gewinde ein metrisches Gewinde M4, M5, M6 oder M8 mit einer Gewindelänge von mindestens 10 mm ist. Durch diese Ausgestaltung der Befestigungseinrichtung kann die medizinische Vorrichtung an vielen Orten stationär befestigt werden, an denen eine Wand- oder Deckenhalterung für einen Flach-bildschirm vorgesehen ist. Eine dem VESA-Standard entsprechende Halterung kann auch an einem Gerätewagen oder einem mobilen oder stationären Regal für einen medizinischen Behandlungsraum vorgesehen sein.

In der Ausnehmung kann ferner ein Kühlkörper zum Abführen von Abwärme der in die medizinische Vorrichtung integrierten Lichtquelle, einer in die medizinische Vorrichtung integrierten Einrichtung zur Leistungsversorgung oder einer anderen in die medizinische Vorrichtung integrierten Einrichtung angeordnet sein. Der Kühlkörper kann mit der Befestigungseinrichtung kombiniert sein. Ferner kann der Kühlkörper mittels der Befestigungseinrichtung in der Ausnehmung an der medizinischen Vorrichtung lösbar befestigt sein. Beispielsweise sind die Gewindelöcher nach VESA an oder nahe bei den Ecken des Kühlkörpers angeordnet. Insbesondere wenn die Befestigungseinrichtung so ausgebildet ist, dass ein Akkumulator oder eine Wand- oder Deckenhalterung, der bzw. die mit der medizinischen Vorrichtung mechanisch verbunden ist, von dem Kühlkörper etwas beabstandet ist (beispielsweise einige mm, insbesondere 2 mm bis 10 mm) schränkt ein an der Befestigungseinrichtung lösbar befestigter Akkumulator oder eine an der Befestigungseinrichtung befestigte Wand- oder Deckenhalterung eine Luftströmung durch den Kühlkörper und dessen Kühlwirkung nur geringfügig ein. Eine Erwärmung des Akkumulators kann sich ferner gerade bei einem modernen Lithium-Ionen-Akkumulator positiv auf die entnehmbare Leistung und die entnehmbare Energie auswirken. Ein Wärmeübertrag an eine Wand- oder Deckenhalterung kann die Kühlwirkung verstärken. Die kombinierte Anordnung der Befestigungseinrichtung und des Kühlkörpers ist somit nicht nur hinsichtlich eines insgesamt geringen Raumbedarfs vorteilhaft.

Bei einer medizinischen Vorrichtung mit einer Befestigungseinrichtung, wie sie hier beschrieben ist, kann die Befestigungseinrichtung eine Taille aufweisen, in die eine schlüssellochförmige Ausnehmung in einer Gehäusewand eines Akkumulators eingehängt werden kann, wobei der Kühlkörper eine Rastnut aufweist, die eine Rastverbindung mit dem Akkumulator ermöglicht. Die Taille an der Befestigungseinrichtung ist ebenso wie die schlüssellochförmige Ausnehmung in der Gehäusewand mit geringem Aufwand herstellbar, ermöglicht aber, gerade in Zusammenwirken mit der Rastverbindung, eine zuverlässige mechanische Verbindung zwischen der medizinischen Vorrichtung und einem Akkumulator, die leicht herstellbar und wieder lösbar ist.

Eine medizinische Vorrichtung, wie sie hier beschrieben ist, kann an der Rückseite des Gehäuses einen Kabelhalter zur Aufbewahrung eines Leistungsversorgungskabels oder eines anderen Kabels umfassen. Herkömmlich ist für Kabel in der Regel ein bestimmter Ort im Transportkoffer vorgesehen. An einem Gerät ist ein Kabelhalter in der Regel nur vorgesehen, wenn das Gerät für eine Verwendung ohne einen Transportkoffer vorgesehen ist. Es hat sich jedoch herausgestellt, dass es auch bei einer medizinischen Vorrichtung, die zum Transport in einem Transportkoffer vorgesehen und ausgebildet ist, vorteilhaft ist, wenn Kabel an einem Kabelhalter an der medizinischen Vorrichtung selbst aufbewahrt werden können. Insbesondere kann unmittelbar nach dem Entnehmen der medizinischen Vorrichtung aus dem Transportkoffer dieser wieder geschlossen werden, um beispielsweise die medizinische Vorrichtung außen an dem Transportkoffer zu befestigen. Erst danach kann dann das Kabel von dem Kabelhalter an der medizinischen Vorrichtung genommen werden. Das Kabel muss deshalb nicht zwischenzeitlich abgelegt werden, wobei es verlegt oder beschädigt werden könnte. Der Kabelhalter an der Rückseite des Gehäuses der medizinischen Vorrichtung ermöglicht somit einen schnelleren und präziseren Arbeitsablauf beim Aufstellen bzw. Inbetriebnehmen der medizinischen Vorrichtung.

Einige Merkmale der hierin beschriebenen medizinischen Vorrichtungen, insbesondere die Neigung der Frontfläche mit dem Bildschirm zur Rückseite hin und die beschriebene Anordnung der Lichtquelle und/oder der Einrichtung zur Leistungsversorgung, ermöglichen eine tiefe Lage des Masseschwerpunkts der medizinischen Vorrichtung. Insbesondere weist der Massenschwerpunkt einen Abstand von der Bodenfläche des Gehäuses auf, der höchstens ein Drittel oder höchstens ein Viertel der Gesamthöhe des Gehäuses beträgt. Dies ermöglicht eine besonders hohe Standfestigkeit der medizinischen Vorrichtung und verringert das Risiko einer Beschädigung durch Umkippen.

Eine medizinische Vorrichtung, wie sie hier beschrieben ist, weist insbesondere eine teilweise in das Gehäuse integrierte Halteeinrichtung zum Halten eines Gefäßes für ein bei einer endoskopischen Untersuchung zu verwendendes Fluid auf. Derartige Halteeinrichtungen, beispielsweise für Flaschen aus Glas oder Kunststoff, stehen herkömmlich vom Gehäuse ab, beispielsweise in Form eines Korbs oder eines Bechers. Zur Aufnahme der resultierenden Kräfte und Momente muss eine herkömmliche Halteeinrichtung entsprechend schwer dimensioniert sein und weist eine entsprechend hohe Masse auf. Durch die zumindest teilweise Integration in das Gehäuse ist eine Ausführung mit geringer Masse möglich. Ferner reduziert die zumindest teilweise Integration der Halteeinrichtung in das Gehäuse das Risiko einer Beschädigung der Halteeinrichtung und/oder des Gefäßes.

Ein Akkumulator für eine medizinische Vorrichtung zur Unterstützung einer endoskopischen Untersuchung umfasst eine Gehäusewand, eine schlüssellochförmige Ausnehmung in der Gehäusewand, zur mechanischen Kopplung des Akkumulators mit einer Befestigungseinrichtung der medizinischen Vorrichtung und eine Rasteinrichtung zum Eingreifen in eine Rastnut an der medizinischen Vorrichtung.

### Kurzbeschreibung der Figuren

Nachfolgend werden beispielhafte Ausführungsformen anhand der beigefügten Figuren näher erläutert. Es zeigen:
- Figur 1: eine schematische axonometrische Darstellung einer medizinischen Vorrichtung von vorn;
- Figur 2: eine schematische axonometrische Darstellung der medizinischen Vorrichtung von hinten;
- Figur 3: eine schematische axonometrische Darstellung der medizinischen Vorrichtung von links;
- Figur 4: eine schematische axonometrische Darstellung der medizinischen Vorrichtung von rechts;
- Figur 5: eine weitere schematische axonometrische Darstellung der medizinischen Vorrichtung von hinten;
- Figur 6: eine schematische axonometrische Darstellung der medizinischen Vorrichtung von oben;
- Figur 7: eine schematische axonometrische Darstellung der medizinischen Vorrichtung aus einer raumdiagonalen Richtung;
- Figur 8: eine weitere schematische axonometrische Darstellung der medizinischen Vorrichtung aus einer raumdiagonalen Richtung;
- Figur 9: schematische Darstellungen einer Befestigungseinrichtung für einen Akkumulator der medizinischen Vorrichtung;
- Figur 10: schematische Darstellungen einer Befestigungseinrichtung zum lösbaren Befestigen der medizinischen Vorrichtung an einem Transportkoffer;
- Figur 11: eine schematische Darstellung eines Transportkoffers für eine medizinische Vorrichtung;
- Figur 12: eine weitere schematische Darstellung des Transportkoffers;
- Figur 13: eine weitere schematische Darstellung des Transportkoffers mit der medizinischen Vorrichtung.

### Beschreibung der Ausführungsformen

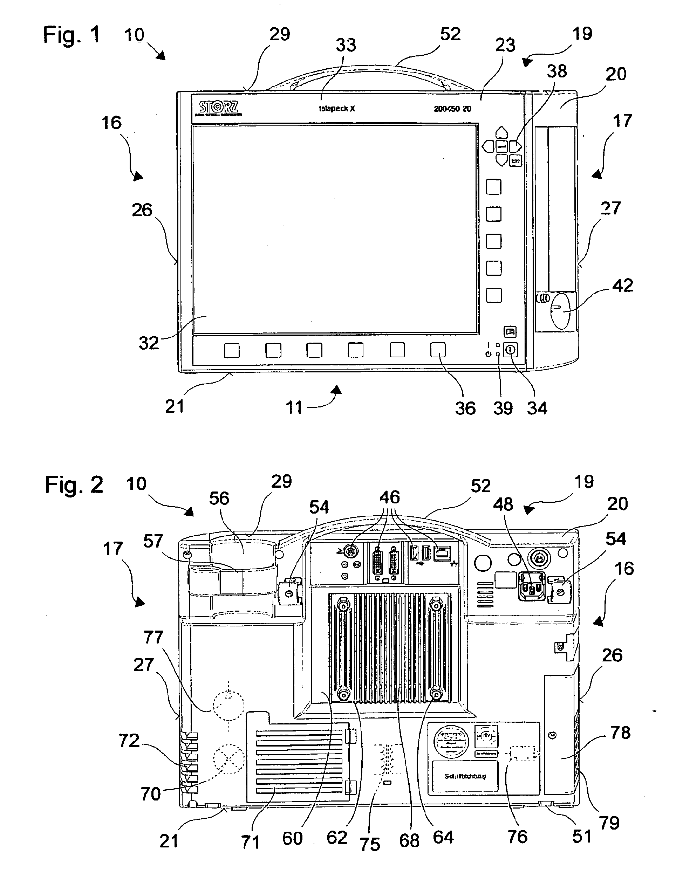

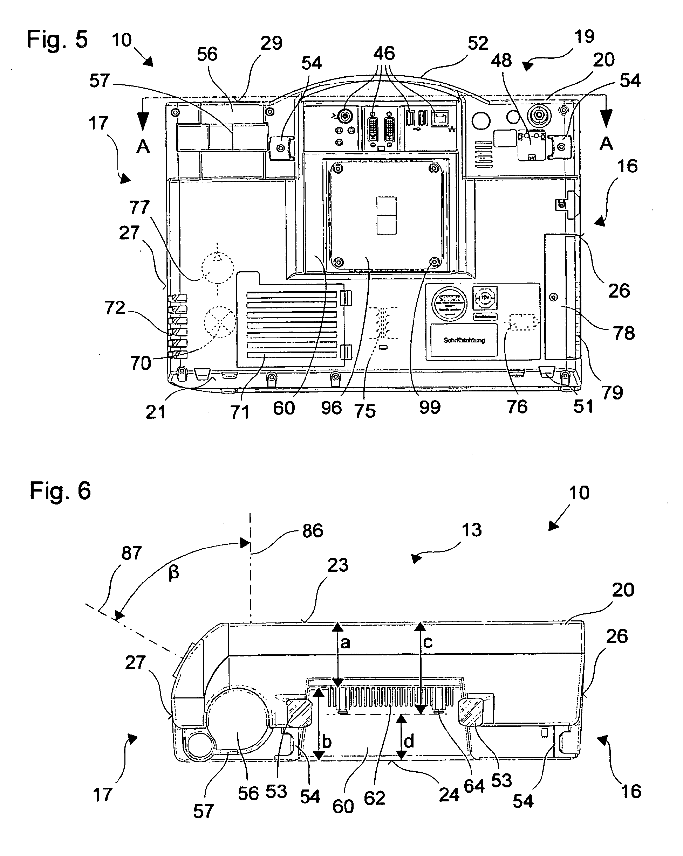

Die Figuren 1 bis 8 zeigen schematische axonometrische Darstellungen einer medizinischen Vorrichtung 10 aus verschiedenen Perspektiven bzw. Blickrichtungen. Figur 6 zeigt eigentlich eine Darstellung eines Schnitts entlang der in Figur 5 gezeigten Ebene A-A, die jedoch nur einen Handgriff schneidet, so dass darunterliegende Merkmale besser sichtbar sind. Die nachfolgend verwendeten Richtungsangaben "vom", "hinten", "links", "rechts", "oben" und "unten" beziehen sich dabei auf die vorgesehene Verwendung der medizinischen Vorrichtung 10, bei der sie gewöhnlich so auf einer festen horizontalen Unterlage steht, dass medizinisches Personal mit Benutzerschnittstelle an der Front der medizinischen Vorrichtung optimal interagieren kann.

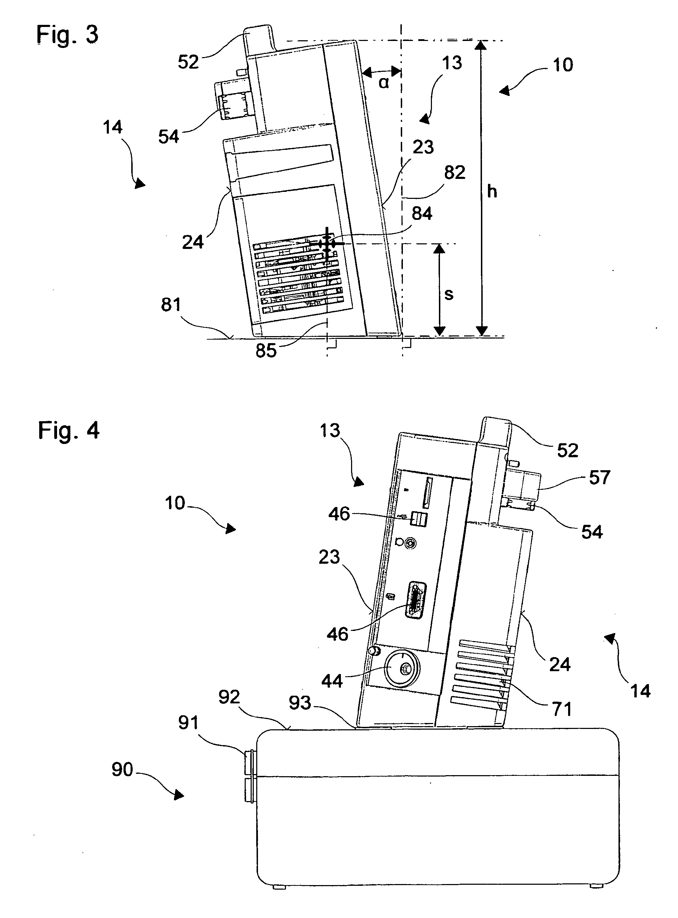

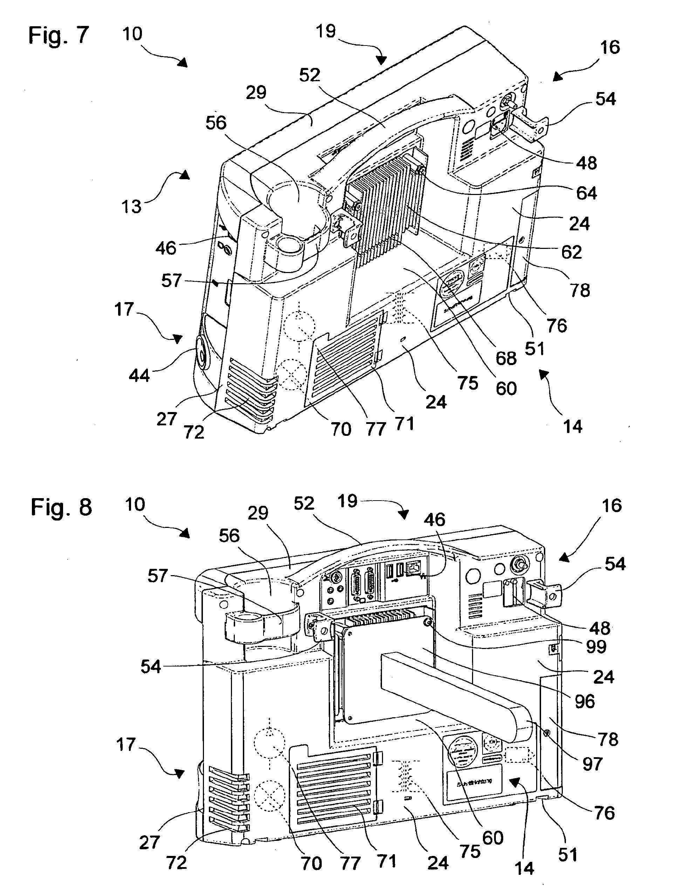

Die medizinische Vorrichtung 10 weist eine Unterseite 11, eine Vorderseite bzw. Front 13, eine Rückseite 14, eine linke Seite 16, eine rechte Seite 17 und eine Oberseite 19 auf. Die linke Seite 16 ist von einer Person, welche die medizinische Vorrichtung 10 bedient bzw. verwendet, aus gesehen links. Die Front 13 der medizinischen Vorrichtung 10 ist insbesondere in Figur 1 dargestellt, die Rückseite 14 insbesondere in den Figuren 2 und 5. Die linke Seite 16 der medizinischen Vorrichtung 10 ist insbesondere in Figur 3 dargestellt, die rechte Seite 17 in Figur 4. Figur 6 zeigt die medizinische Vorrichtung 10 von oben, die Figuren 7 und 8 zeigen jeweils zumindest Teile der Rückseite 14, der rechten Seite 17 und der Oberseite 19.

Die medizinische Vorrichtung 10 umfasst ein Gehäuse 20 mit einer Bodenfläche 21, einer Frontfläche 23, einer Rückseitenfläche 24, einer linke Seitenfläche 26, einer rechten Seitenfläche 27 und einer Oberseitenfläche 29. Die Frontfläche 23 des Gehäuse 20 ist im Wesentlichen eben und umfasst einen rechteckigen Bildschirm 32 bzw. dessen transparente Oberfläche, der von einem L-förmigen, U-förmigen oder rechteckigen Rahmen 33 an zwei, drei oder allen Seiten umgeben ist. In dem Rahmen 33 sind ein Ein-/Ausschalter 34, mehrere Tasten 36 einschließlich Cursortasten 38 und Kontrollleuchten 39, die beispielsweise den Betriebsstatus der medizinischen Vorrichtung 10 anzeigen, angeordnet. Abweichungen der räumlichen Gestalt der Frontfläche 23 von einer Ebene können von dem Bildschirm 32, dem Ein-/Ausschalter 34, den Tasten 36, den Cursortasten 38 und den Kontrollleuchten 39 herrühren, die jeweils gegenüber dem Rahmen 33 flächenbündig, erhaben oder versenkt angeordnet sein können. Diese Abweichungen betragen jedoch bei dem hier dargestellten Ausführungsbeispiel nur wenige Millimeter, insbesondere weniger als 5 mm.

Die Rückseitenfläche 24 nimmt, wie insbesondere in den Figuren 2, 5, 7 und 8 erkennbar ist, U-förmig einen Großteil der Rückseite 14 des Gehäuses 20 ein. Die Rückseitenfläche 24 ist im Wesentlichen eben, Abweichungen von einer ideal ebenen Gestalt liegen dort vor, wo Schrift oder andere Zeichen erhaben oder vertieft vorliegen, sowie bei nachfolgend beschriebenen Klappen, Belüftungsgittern und Schraubenlöchern. Diese Abweichungen betragen jedoch nur wenige Millimeter, insbesondere weniger als 5 mm, und/oder nehmen nur kleine Teilflächen ein. Neben der im Wesentlichen ebenen Rückseitenfläche 24 weist die Rückseite 14 der medizinischen Vorrichtung 10 bzw. von dessen Gehäuse 20 jedoch auch weitere Flächen auf, die gegenüber der dominanten Rückseitenfläche 24 zurückgesetzt sind. Dies ist insbesondere in den Figuren 7 und 8 erkennbar.

Die linke Seitenfläche 26 des Gehäuses 20 ist in Figur 3 dargestellt. Insbesondere in Figur 6 ist erkennbar, dass die linke Seitenfläche 26 des Gehäuses 20 im Wesentlichen eben und näherungsweise im rechten Winkel zur Frontfläche 23 und zur Rückseitenfläche 24 angeordnet ist. Die rechte Seitenfläche 27 ist in Figur 4 dargestellt. Insbesondere in Figur 6 ist erkennbar, dass die rechte Seitenfläche 27 einen gewölbten Abschnitt aufweist, der mit einem Winkel deutlich kleiner als 90 Grad in die Frontfläche 23 übergeht.

In dem gewölbten Abschnitt der rechten Seitenfläche 27 des Gehäuses 20 ist eine Kopplungseinrichtung 42 angeordnet (vgl. Figur 1), in die eine Adaptereinrichtung 44 eingesetzt werden kann (vgl. Figuren 4, 7). Über die Adaptereinrichtung 44 kann eine Kupplung an einem Ende eines Lichtleitkabels mit der Kopplungseinrichtung 42 gekoppelt werden, um Licht von der medizinischen Vorrichtung zu einem Endoskop zu übertragen. Nach Austausch der Adaptereinrichtung 44 gegen eine andere, in den Figuren nicht dargestellte Adaptereinrichtung, kann eine Kopplung an einem proximalen Ende eines Endoskops unmittelbar über diese andere Adaptereinrichtung mit der Kopplungseinrichtung 42 gekoppelt werden.

Insbesondere in den Figuren 2, 4, 5 und 8 ist erkennbar, dass die medizinische Vorrichtung 10 zahlreiche Signalanschluss-Steckbuchsen unterschiedlichen Formats (unter anderem USB, Ethernet, DVI und andere digitale und analoge Schnittstellen) und eine Leistungsversorgungs-Steckbuchse 48 aufweist. Die Leistungsversorgungs-Steckbuchse 48 ist, wenn die medizinische Vorrichtung für den europäischen Markt vorgesehen ist, beispielsweise eine Buchse für einen Kaltgerätestecker zum Anschluss an ein elektrisches Leistungsversorgungsnetz mit beispielsweise 230 Volt und 50 Hertz.

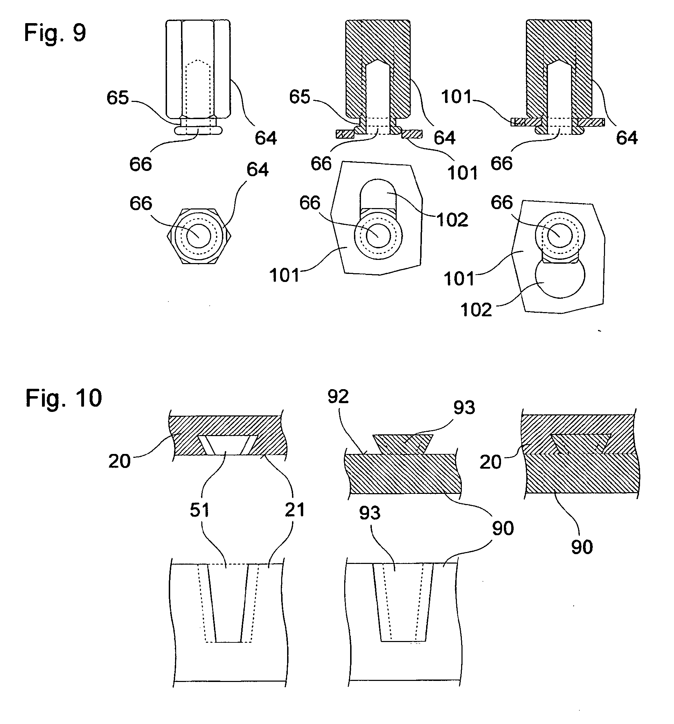

Insbesondere in den Figuren 2, 5 und 8 ist erkennbar, dass das Gehäuse 20 der medizinischen Vorrichtung 10 an der Bodenfläche 21 mehrere Befestigungsvorrichtungen 51 aufweist, die unten anhand der Figur 10 detaillierter dargestellt werden.

In den Figuren 1 bis 8 ist erkennbar, dass die medizinische Vorrichtung 10 an ihrer Oberseite 19 einen bügelförmigen, insbesondere bogenförmigen, Handgriff 52 zum Tragen der medizinischen Vorrichtung aufweist, der gegenüber der Oberseitenflächen 29 des Gehäuses 20 übersteht, jedoch mit dem Gehäuse 20 oder einem Teil des Gehäuses 20 einstückig ausgebildet ist. Da die Figur 6, wie bereits oben erwähnt, einen Schnitt entlang der in Figur 5 gezeigten Ebene A-A darstellt, sind in Figur 6 lediglich Querschnitte 53 des Handgriffs 52 erkennbar. Dadurch sind in Figur 6 unter dem Handgriff 52 liegende Merkmale der medizinischen Vorrichtung 10 erkennbar.

Insbesondere in den Figuren 2 und 5 bis 8 ist erkennbar, dass die medizinische Vorrichtung 10 zwischen dem oberen Rand der Rückseitenfläche 24 des Gehäuses 20 und dem Handgriff 52 ein Paar hakenförmiger Kabelhalter 54 aufweist, auf die ein oder mehrere Kabel aufgewickelt werden können, wenn diese nicht benötigt werden. In den gleichen Figuren ist erkennbar, dass die medizinische Vorrichtung 10 eine Halteeinrichtung 56, 57 für eine Flasche oder ein anderes Gefäß für ein bei einer endoskopischen Untersuchung zu verwendendes Fluid aufweist. Die Halteeinrichtung umfasst, wie insbesondere in den Figuren 6 bis 8 erkennbar ist, einen bogenförmigen Bügel 57, der ein kreiszylindrisches Volumen teilweise umschließt, das zu einem anderen Teil durch eine von der Rückseite und von der Oberseite ausgehende Ausnehmung 56 im Gehäuse 20 gebildet wird.

Insbesondere in den Figuren 7 und 8 ist erkennbar, dass die Rückseitenfläche 24 des Gehäuses 20 U-förmig näherungsweise drei Seiten einer Ausnehmung 60 an der Rükseite des Gehäuses 20 umfasst. In der Ausnehmung 60 sind ein im Wesentlichen rechteckiger Kühlkörper 62 zum Abführen von Abwärme und an den vier Ecken des Kühlkörpers 62, vier unten anhand der Figur 9 näher beschriebene Stifte, die eine Befestigungseinrichtung 64 bilden, angeordnet. Zwischen dem Kühlkörper 62 und dem Handgriff 52 sind einige der bereits erwähnten Signalanschluss-Steckbuchsen 46 angeordnet. Die Ausnehmung wird nach oben teilweise vom Handgriff 52 abgeschlossen.

Im Gehäuse 20 der medizinischen Vorrichtung 10 ist eine in den Figuren 2, 5, 7 und 8 angedeutete Lichtquelle 70 angeordnet. Die Lichtquelle 70 kann durch eine Wartungsklappe 71 gewartet werden. Insbesondere kann ein Leuchtmittel (Halogenlampe, Hochdruck-Gasentladungslampe, Leuchtdioden etc.) bei geöffneter Wartungsklappe 71 ausgetauscht werden. An der rechten Seitenfläche 27 ist ein Lüftungsgitter 72 für die Lichtquelle 70 angeordnet. Auch in der Wartungsklappe 71 kann ein Lüftungsgitter angeordnet sein.

Die medizinische Vorrichtung 10 umfasst ferner ein in den Figuren 2, 5, 7 und 8 ebenfalls nur angedeutetes Netzteil 75, das beispielsweise einen Transformator und elektronische Schaltungen zum Erzeugen und Glätten von einer oder mehreren Gleichspannungen zur Versorgung der Lichtquelle 70 und anderer Bestandteile und Komponenten der medizinischen Vorrichtung 10 umfasst.

Ferner umfasst die medizinische Vorrichtung eine in den Figuren 2, 5, 7 und 8 angedeutete Pumpe 77 zum Pumpen eines Fluids, insbesondere von Luft zu der Kopplungseinrichtung 42. Mit einer Pumpe 77 ist hier auch ein Verdichter oder ein Gebläse oder eine andere Einrichtung zum Forded eines kompressiblen oder inkompressiblen Fluids gemeint. Ein Schalldämpfer 76 an der Saugseite bzw. am Einlass der Pumpe 77 ist über eine Wartungsklappe 78 mit einem Lüftungsgitter 79 bzw. Lüftungsöffnungen zugänglich. Der Schalldämpfer 76 kann ein Filter zur Filterung des von der Pumpe 77 über den Schalldämpfer 76 angesaugten Fluids umfassen.

Insbesondere in den Figuren 3 und 4 ist erkennbar, dass die Frontfläche 23 und die Rückseitenfläche 24 nicht im rechten Winkel zur Bodenfläche 21 angeordnet sind. Stattdessen sind die Frontfläche 23 und die Rückseitenfläche 24 zur Rükseite 14 der medizinischen Vorrichtung 10 hin geneigt. Zur Verdeutlichung sind in Figur 3 eine Oberfläche 81 einer Tischplatte oder eine andere ebene Oberfläche, auf der die medizinische Vorrichtung 10 steht, sowie ein Lot 82 auf die Oberfläche 81 gezeigt. Der Winkel *α* zwischen der Frontfläche 23 und dem Lot 82 beträgt bei diesem Ausführungsbeispiel zwischen 8 Grad und 9 Grad, insbesondere 8,3 Grad.

In Figur 3 ist außerdem der Massenschwerpunkt 84 der medizinischen Vorrichtung 10 gezeigt. Vor allem aufgrund der Neigung der Frontfläche 23 und des verhältnismäßig schweren Bildschirms 32 darin sowie aufgrund der tiefen Anordnung der Lichtquelle 70, des Netzteils 75 und der Pumpe 77 ist eine Position des Schwerpunkts 84 mit geringem Abstand von der Bodenfläche 21 erzielbar. Insbesondere beträgt der Abstand s des Massenschwerpunkts 84 von der Bodenfläche 21 höchstens ein Drittel, besser noch höchstens ein Viertel der Höhe h (gemessen senkrecht zur Bodenfläche 21) des Gehäuses 20 (ohne den Handgriff 52). Ferner beträgt der Abstand des Schnittpunkts des Lots durch den Schwerpunkt 84 auf die Bodenfläche 21 von den Schnittlinien der durch die Bodenfläche 21 definierten Ebene mit den durch die Frontfläche 23 bzw. die Rückseitenfläche 24 definierten Ebenen jeweils mindestens ein Drittel des parallel zur Bodenfläche 21 gemessenen Abstands der Frontfläche 23 und der Rückseitenfläche 24.

Zur Erzielung dieser Schwerpunktlage sind die Lichtquelle 70, das Netzteil 75 und die Pumpe 77 so angeordnet, dass die von ihnen eingenommenen Raumbereiche sich jeweils nur über die untere Hälfte oder weniger, besser noch über die unteren zwei Fünftel oder weniger der Höhe h des Gehäuses 20 erstrecken.

In Figur 6 sind das Lot 86 auf die Frontfläche 23 des Gehäuses 20 und eine Richtung 87 der Kopplungseinrichtung 42 dargestellt. Die Richtung 87 der Kopplungseinrichtung 42 ist die Richtung, in der eine Kupplung eines Lichtleitkabels oder eine Kupplung an einem proximalen Ende eines Endoskops in eine Adaptereinrichtung 44 in der Kopplungseinrichtung 42 eingeführt werden kann. Der Winkel *ß* zwischen der Richtung 87 und dem Lot 86 beträgt ca. 60 Grad.

In Figur 4 ist die medizinische Vorrichtung 10 mit einem Transportkoffer 90 zum geschützten Transport der medizinischen Vorrichtung 10 gezeigt. Der Transportkoffer 90 weist einen Verschluss 91 zum Verschließen auf. An einer Außenfläche 92 des Transportkoffers 90 sind zu den Befestigungseinrichtungen 51 an der Bodenfläche 21 komplementäre Befestigungseinrichtungen 93 angeordnet. Die Befestigungseinrichtungen 93 am Transportkoffer 90 können bündig mit der Außenfläche 92 abschließen oder, wie in Figur 4 angedeutet, gegenüber dieser teilweise nach außen überstehen. Ein Beispiel für eine Befestigungseinrichtung 51 an der Bodenfläche 21 des Gehäuses 20 der medizinischen Vorrichtung 10 und eine komplementäre Befestigungseinrichtung 93 am Transportkoffer 90 wird unten mit Bezug auf Figur 10 dargestellt.

In den Figuren 5 und 8 ist die medizinische Vorrichtung 10 an einem Träger mit einer Platte 96 und einem Arm 97 befestigt dargestellt. Der Träger 96, 97 ist beispielsweise Teil einer Wand- oder Deckenhalterung. Die Platte 96 weist vier Durchgangsbohrungen auf, die dem VESA-Standard für Wand- und Deckenhalterungen von Flachbildschirmen entsprechen. Je eine Schraube 99 ist durch eine dieser Durchgangsbohrungen in der Platte 96 in ein Gewindeloch 66 bzw. eine Bohrung mit Innengewinde der Befestigungseinrichtung 64 geschraubt, wobei in Figur 8 nur eine der Schrauben 99 dargestellt ist. Alternativ kann an der Befestigungseinrichtung 64 ein Akkumulator lösbar befestigt werden. Dazu ist ferner eine Rastnut 68 in Form von U-förmigen Ausnehmungen oder Kerben in mehreren Rippen des Kühlkörpers 62 ausgebildet, die insbesondere in den Figuren 2 und 7 erkennbar ist. Dies ist unten anhand der Figur 9 näher beschrieben.

Figur 9 zeigt schematische Darstellungen eines der vier gleichen Bestandteile der Befestigungseinrichtung 64 in der Ausnehmung 60. Die Befestigungseinrichtung 64 weist eine Taille 65 bzw. einen Abschnitt mit vermindertem Querschnitt und eine Gewindebohrung 66 auf. In die Gewindebohrung 66 kann eine Schraube eingesetzt werden, um die Befestigungseinrichtung 64 an der oben anhand der Figuren 5 und 8 dargestellten Platte 96 des Trägers 95 festzuschrauben. Alternativ kann eine Gehäusewand 101 eines Akkumulators, von der in Figur 9 nur ein kleiner Ausschnitt dargestellt ist, mit einer schlüssellochförmigen Ausnehmung 102 in die Befestigungseinrichtung 64 eingehängt werden. In Figur 9 in der Mitte ist dargestellt, wie die schlüssellochformige Öffnung 102 über die Befestigungseinrichtung 64 geführt wird, rechts ist die Gehäusewandung 101 in die Befestigungseinrichtung 64 eingehängt gezeigt. Dabei ist in der Mitte und rechts jeweils oben ein Schnitt parallel zur Achse der Befestigungseinrichtung 64 und unten eine Draufsicht in Richtung parallel zu der Achse der Befestigungseinrichtung 64 gezeigt.

An der dem Kühlkörper 62 und den Befestigungseinrichtungen 64 zugewandten Gehäusewand 101 des Akkumulators kann eine Rastnase angeordnet sein. In der in Figur 9 rechts dargestellten Position der Gehäusewand 101 relativ zu den Befestigungseinrichtungen 64 greift die Rastnase des Akkumulators in die in den Figuren 2 und 7 erkennbare Rastnut 68 ein. Der Akkumulator wird damit rastend in der in Figur 9 rechts dargestellten Position gehalten werden, um ein unbeabsichtigtes Lösen von den Befestigungseinrichtungen 64 zu verhindern. Zum Abnehmen des Akkumulators von den Befestigungseinrichtungen 64 wird die Rastung beispielsweise durch eine entsprechende Kraft überwunden. Altemativ ist an dem Akkumulator ein Hebel oder eine andere Einrichtung vorgesehen, mit der die Rastnase des Akkumulators aus der Rastnut 68 gezogen werden kann.

Bei der oben anhand der Figuren 2 und 5 bis 9 dargestellten Ausführungsform hält die Befestigungseinrichtung 64 in der Ausnehmung 60 gleichzeitig den Kühlkörper 62. Insbesondere ist die Befestigungseinrichtung 64 in Form sechseckiger Säulen ausgeführt, die mittels eines dazugeeigneten Werkzeugs an das Gehäuse 20 der medizinischen Vorrichtung 10 geschraubt sind. Die Befestigungseinrichtung 64 kann damit gleichzeitig den Kühlkörper 62 halten, indem die Schraubverbindungen zwischen den Säulen der Befestigungseinrichtung 64 und dem Gehäuse durch Öffnungen bzw. Durchgangslöcher in dem Kühlkörper hindurch greifen. Altemativ ist der Kühlkörper abweichend von der Darstellung in den Figuren 2 und 5 bis 9 lösbar an der Befestigungseinrichtung befestigt. Dabei können die Befestigungseinrichtung und der Kühlkörper so ausgebildet sein, dass der Kühlkörper und ein Akkumulator Alternativ oder gleichzeitig lösbar an der Befestigungseinrichtung in der Ausnehmung befestigt werden können.

Die Figur 10 zeigt links und in der Mitte schematische Darstellungen der Befestigungseinrichtung 51 an der Bodenfläche 21 des Gehäuses 20 der medizinischen Vorrichtung bzw. der Befestigungseinrichtung 93 an einer Außenfläche 92 des Transportkoffers 90. Dabei sind jeweils oben ein Querschnitt und unten eine Draufsicht dargestellt. Rechts in Figur 10 ist ein Querschnitt der miteinander mechanisch verbundenen Befestigungseinrichtungen 51, 93 dargestellt, über die die Bodenfläche 21 des Gehäuses 20 der medizinischen Vorrichtung 10 mechanisch mit dem Transportkoffer 90 verbunden ist.

Die Befestigungseinrichtung 51 ist eine Nut mit einem schwalbenschwanzförmigen bzw. trapezförmigen Querschnitt, wobei der Querschnitt sich von einem Ende der Nut zum anderen verringert. Die Befestigungseinrichtung 93 an der Außenfläche 92 des Transportkoffers 90 ist ein Steg mit einem schwalbenschwanzförmigen bzw. trapezförmigen Querschnitt, der sich von einem Ende zum anderen Ende des Stegs verringert. Die Querschnitte der Nut 51 und des Stegs 53 sind aneinander angepasst bzw. komplementär zueinander. Wenn, wie in Figur 10 rechts dargestellt, der Steg 93 in die Nut 51 geschoben ist, liegt eine in Richtung senkrecht zur Bodenfläche 21 formschlüssige mechanische Verbindung zwischen dem Gehäuse 20 der medizinischen Vorrichtung 10 und dem Transportkoffer 90 vor.

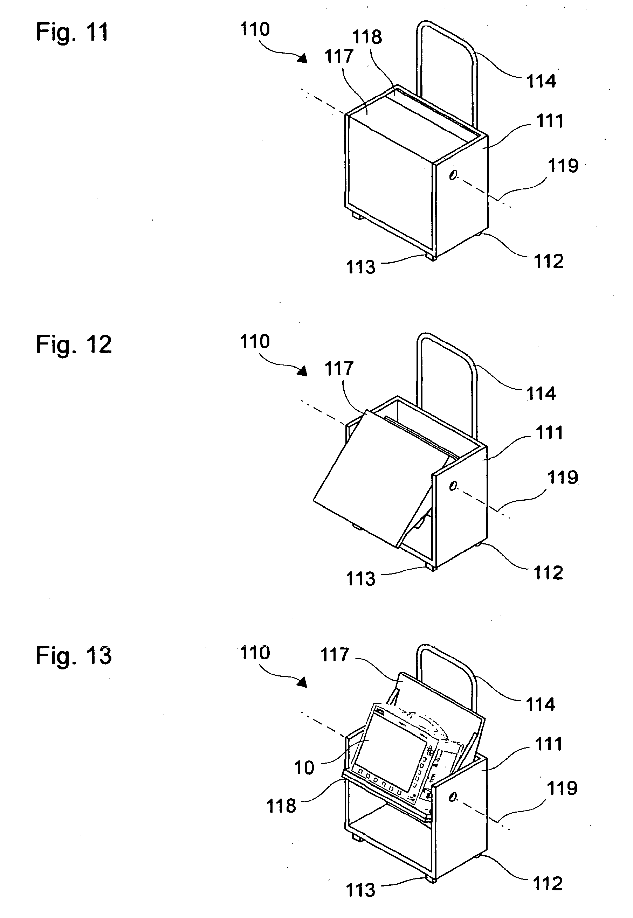

Die Figuren 11, 12 und 13 zeigen schematische axonometrische Darstellungen eines Transportkoffers 110 für die oben anhand der Figuren 1 bis 8 dargestellte medizinische Vorrichtung 10. Der Transportkoffer 110 weist eine erste Schale 111 auf, die vier im wesentlichen ebene und im wesentlichen rechteckige Abschnitte umfasst. Nahe vier benachbarten und im Wesentlichen in einer Ebene liegenden Ecken der ersten Schale sind zwei Räder 112 und zwei starre Füße 113 angeordnet, auf denen die erste Schale 111 auf einem ebenen Untergrund abgestellt werden kann. Von den beiden Rädern 112, die um die gleiche Achse rotieren können, ist in den Figuren 11 bis 13 nur jeweils eines sichtbar. Wenn der Transportkoffer mit den beiden Rädern 112 und den beiden Füßen 113 auf einem ebenen Untergrund steht, der von der Horizontalen nicht zu stark abweicht, verhindert die Haftreibung zwischen den starren Füßen 113 und dem Untergrund eine Bewegung des Transportkoffers 110 parallel zu dem Untergrund.

Ferner ist ein starrer oder teleskopisch ausziehbarer bügelförmiger Griff 114 an einem rechteckigen Abschnitt der ersten Schale 111 angeordnet, nahe dessen vom Griff 114 abgewandten Rand die beiden Räder 112 angeordnet sind. Mittels des Griffs 114 kann der Transportkoffer 110 so gekippt werden, dass er nur noch mit den Rädern 112, aber nicht mehr mit den Füßen 113 auf dem Untergrund aufliegt, und dann mit geringem Rollwiderstand über den Untergrund gezogen oder geschoben werden.

Ferner weist der Transportkoffer 110 eine zweite Schale 117 auf, die zwei L-förmig aneinander grenzende, im wesentlichen ebene und im wesentlichen rechteckige Abschnitte umfasst. Ein streifenförmiger Abschnitt 118 am Rand der zweiten Schale 117 ist gegenüber dem Rest der zweiten Schale 117 bewegbar. Die zweite Schale 117 ist gegenüber der ersten Schale 111 um eine Achse 119 schwenkbar. Bei der in Figur 11 gezeigten Position der zweiten Schale 117 verschließt die zweite Schale 117 eine entsprechend geformte Öffnung in der ersten Schale 111, und die erste Schale 111 und die zweite Schale 117 bilden ein geschlossenes Behältnis.

Nach einem Umklappen des streifenförmigen Abschnitts 118 kann die zweite Schale 117 um die Achse 119 geschwenkt werden, von der in Figur 11 gezeigten Position über die in Figur 12 gezeigte Position bis zu der in Figur 13 gezeigten Position.

Die oben anhand der Figuren 1 bis 8 und 11 bis 13 dargestellte medizinische Vorrichtung 10 kann innen an der L-förmigen zweiten Schale 117 lösbar befestigt werden, beispielsweise ähnlich wie oben anhand der Figur 10 dargestellt. Die medizinische Vorrichtung 10 kann ständig an der zweiten Schale 117 befestigt bleiben, sowohl während eines Transports als auch bei ihrer Verwendung bei einer Endoskopie. Während des Transports ist die zweite Schale 117 in der in Figur 11 gezeigten Position verriegelt. Die erste Schale 111 und die zweite Schale 117 umschließen die medizinische Vorrichtung 10 vollständig und schützen sie vor Umwelteinflüssen. Nach einem Schwenken bzw. Rotieren der zweiten Schale 117 um die Achse 119 in die in Figur 13 dargestellte Position ist die medizinische Vorrichtung 10 unmittelbar einsetzbar ohne von der zweiten Schale 117 abgenommen werden zu müssen.

Anhand der Figuren 3 bzw. 5 und 8 bzw. 11 bis 13 wurde die Befestigung der medizinischen Vorrichtung 10 an einer Außenfläche 92 eines Transportkoffers 90 bzw. an einer Platte 96 einer Wand- oder Deckenhalterung bzw. in einem Transportkoffer 110 beschrieben. Alternativ oder zusätzlich kann die medizinische Vorrichtung 10 ausgebildet sein, um mittels der Befestigungseinrichtungen 51 an ihrer Bodenfläche und/oder mittels der Befestigungseinrichtungen 64 in ihrer Ausnehmung 60 an einem Fachboden oder einer Befestigungseinrichtung gemäß dem VESA-Standard an einem mobilen oder stationären Regal oder Geräteträger oder an einer Sockelplatte befestigt zu werden.

### Bezugszeichen

- 10: Medizinische Vorrichtung
- 11: Unterseite der medizinischen Vorrichtung 10
- 13: Vorderseite bzw. Front der medizinischen Vorrichtung 10
- 14: Rükseite der medizinischen Vorrichtung 10
- 16: linke Seite der medizinischen Vorrichtung 10
- 17: rechte Seite der medizinischen Vorrichtung 10
- 19: Oberseite der medizinischen Vorrichtung 10
- 20: Gehäuse der medizinischen Vorrichtung 10
- 21: Bodenfläche des Gehäuses 20
- 23: Frontfläche des Gehäuses 20
- 24: Rückseitenfläche des Gehäuses 20
- 26: linke Seitenfläche des Gehäuses 20
- 27: rechte Seitenfläche des Gehäuses 20
- 29: Oberseitenfläche des Gehäuses 20
- 32: Bildschirm
- 33: Rahmen
- 34: Ein-/Ausschalter
- 36: Taste
- 38: Cursortaste
- 39: Kontrollleuchte
- 42: Kopplungseinrichtung
- 44: Adaptereinrichtung
- 46: Signalanschluss-Steckbuchse
- 48: Leistungsversorgungs-Steckbuchse
- 51: Befestigungseinrichtung an der Bodenfläche 21
- 52: Handgriff an der Oberseitenfläche 29
- 53: Querschnitt des Handgriffs 52
- 54: Kabelhalter
- 56: halbzylindrische Ausnehmung
- 57: Halteeinrichtung für Flasche
- 60: Ausnehmung an der Rückseite des Gehäuses 20
- 62: Kühlkörper in der Ausnehmung 60
- 64: Befestigungseinrichtung
- 65: Taille an Befestigungseinrichtung 64
- 66: Gewindebohrung in Befestigungseinrichtung 64
- 68: Rastnut im Kühlkörper 62
- 70: Lichtquelle
- 71: Wartungsklappe für Lichtquelle 70
- 72: Lüftungsgitter für Lichtquelle 70
- 75: Netzteil
- 76: Schalldämpfer der Pumpe 77
- 77: Pumpe
- 78: Wartungsklappe für Pumpe 77
- 79: Lüftungsgitter in Wartungsklappe 78
- 81: Oberfläche einer Tischplatte
- 82: Lot auf die Oberfläche 81
- 84: Massenschwerpunkt der medizinischen Vorrichtung 10
- 85: Lot durch Massenschwerpunkt 84 auf Oberfläche 81
- 86: Lot auf die Frontfläche 23 des Gehäuses 20
- 87: Richtung der Kopplungseinrichtung 42
- 90: Transportkoffer für die medizinische Vorrichtung 10
- 91: Verschluss am Transportkoffer 90
- 92: Außenfläche des Transportkoffers 90
- 93: Befestigungseinrichtung am Transportkoffer 90
- 96: Platte eines Trägers
- 97: Arm des Trägers
- 99: Schraube
- 101: Gehäusewand eines Akkumulators
- 102: schlüssellochförmige Ausnehmung in der Gehäusewand 101
- 110: Transportkoffer
- 111: erste Schale des Transportkoffers 110
- 112: Rad an der ersten Schale 111
- 113: Fuß an der ersten Schale 111
- 114: Griff an der ersten Schale 111
- 117: zweite Schale des Transportkoffers 110
- 118: bewegbarer Abschnitt an der zweiten Schale 117
- 119: Achse

## Patentansprüche

1. Medizinische Vorrichtung (10) zur Unterstützung einer endoskopischen Untersuchung, mit:
einem **Gehäuse** (20) mit einer Bodenfläche (21), einer Frontfläche (23) und einer Rückseitenfläche (24), die jeweils im Wesentlichen eben ausgebildet sind;
einer **Lichtquelle** (70), die in dem Gehäuse (20) angeordnet ist, zum Erzeugen von Licht zur Beleuchtung eines mit einem Endoskop zu betrachtenden Gegenstands;
einer **Kopplungseinrichtung** (42) zum unmittelbaren oder mittelbaren Koppeln eines proximalen Endes eines Endoskops an die medizinische Vorrichtung (10), und zum Übertragen von Licht von der medizinischen Vorrichtung (10) zu dem Endoskop;
einem **Bildschirm** (32), der an der Frontfläche (23) des Gehäuses (20) angeordnet ist zum Betrachten eines mittels des Endoskops erfassten Bilds,
**gekennzeichnet dadurch, dass** die Frontfläche (23) den darin integrierten Bildschirm (32) umfasst und
die Frontfläche (23) und die Rückseitenfläche (24) gegenüber der Bodenfläche (21) jeweils um einen **Winkel** zwischen 5 Grad und 15 Grad zum Lot auf die Bodenfläche (21) nach hinten geneigt sind.

2. Medizinische Vorrichtung (10) nach Anspruch 1, bei der die **Kopplungseinrichtung** (42) unter einem Winkel zwischen 50 Grad und 70 Grad zur Frontfläche (23) angeordnet ist.

3. Medizinische Vorrichtung (10) nach einem der vorangehenden Ansprüche, bei der ein von der **Lichtquelle** (70) eingenommener Raumbereich in dem Gehäuse (20) ausschließlich in der an die Bodenfläche (21) des Gehäuses (20) angrenzenden Hälfte angeordnet ist.

4. Medizinische Vorrichtung (10) nach einem der vorangehenden Ansprüche, ferner mit:
einer **Befestigungseinrichtung** (51) an der **Bodenfläche** (21) zum Befestigen des Gehäuses (20) zumindest entweder an einem Transportkoffer (90, 110) für die medizinische Vorrichtung (10) oder an einem Stativ oder an einer anderen Vorrichtung.

5. Medizinische Vorrichtung (10) nach Anspruch 4, ferner mit:
einem Transportkoffer (90, 110), der zur Aufnahme der medizinischen Vorrichtung (10) ausgebildet ist, wobei die Befestigungseinrichtung (93) an dem Transportkoffer (90, 110) und die Befestigungseinrichtung (51) an der Bodenfläche (21) des Gehäuses (20) der medizinischen Vorrichtung (10) für eine lösbare mechanische Verbindung miteinander ausgebildet sind.

6. Medizinische Vorrichtung (10) nach Anspruch 5, bei der die Befestigungseinrichtung (51) an der Bodenfläche (21) des Gehäuses (20) mindestens eine Nut oder einen Steg mit einem Schwalbenschwanz- oder trapezförmigen Querschnitt umfasst, und bei der die Befestigungseinrichtung (93) an dem Transportkoffer (90) komplementär zu der Befestigungseinrichtung (51) an der Bodenfläche (21) des Gehäuses (20) ist und entsprechend mindestens einen Steg oder eine Nut mit **Schwalbenschwanz-** oder trapezförmigem Querschnitt umfasst.

7. Medizinische Vorrichtung (10) nach einem der vorangehenden Ansprüche, bei der die.Rückseitenfläche (24) U-förmig eine **Ausnehmung** (60) umschließt, in der eine Befestigungseinrichtung (64) zur Befestigung eines Akkumulator an der medizinischen Vorrichtung (10) angeordnet ist.

8. Medizinische Vorrichtung (10) nach Anspruch 7, bei der die Befestigungseinrichtung (64) in der Ausnehmung (60) zur **alternativen** oder gleichzeitigen Befestigung eines **Akkumulators** oder eines Kühlkörpers (62) an der medizinischen Vorrichtung (10) oder der medizinischen Vorrichtung (10) an einem **Träger** (96, 97) ausgebildet ist.

9. Medizinische Vorrichtung (10) nach einem der Ansprüche 7 und 8, ferner mit:
einem **Kühlkörper** (62) in der Ausnehmung (60), zum Abführen von Abwärme der Lichtquelle (70), einer in die medizinische Vorrichtung (10) integrierten Leistungsversorgung (75) oder einer anderen Einrichtung (79).

10. Medizinische Vorrichtung (10) nach Anspruch 7, bei der
die Befestigungseinrichtung (64) eine Taille (65) aufweist, in die eine schlüssellochförmigen Ausnehmung (102) in einer Gehäusewand (101) eines Akkumulators eingehängt werden kann,
und insbesondere der Kühlkörper (62) eine Rastnut (68) aufweist, die eine Rastverbindung mit dem Akkumulator ermöglicht.

11. Medizinische Vorrichtung (10) nach einem der vorangehenden Ansprüche, ferner mit:
einem **Kabelhalter** (54) an der Rückseite (14) des Gehäuses (20), zur Aufbewahrung eines Kabels für die medizinische Vorrichtung (10).

12. Medizinische Vorrichtung (10) nach einem der vorangehenden Ansprüche, bei welcher der Abstand (s) des **Massenschwerpunkts** (84) der medizinischen Vorrichtung (10) von der Bodenfläche (21) höchstens ein Drittel der Gesamthöhe (h) des Gehäuses (20) beträgt.

13. Medizinische Vorrichtung (10) nach einem der vorangehenden Ansprüche, ferner mit:
einer **Halteeinrichtung** (56, 57), die teilweise in das Gehäuse (20) integriert ist, zum Halten eines Gefäßes für ein bei einer endoskopischen Untersuchung zu verwendendes Fluid.

## Claims

1. Medical device (10) for supporting an endoscopic examination, comprising:
a **housing** (20) with a base area (21), a front area (23) and a rear side area (24), which each have a substantially planar embodiment;
a **light source** (70), arranged in the housing (20), for generating light for illuminating an object to be observed with an endoscope;
a **coupling means** (42) for direct or indirect coupling of a proximal end of an endoscope to the medical device (10) and for transmitting light from the medical device (10) to the endoscope;
a **screen** (32), arranged on the front area (23) of the housing (20), for observing an image acquired by the endoscope,
**characterized in that**
the front area (23) comprises the screen (32) integrated therein and
the front area (23) and the rear side area (24) are inclined backward in relation to the base area (21), respectively by an **angle** of between 5 degrees and 15 degrees with respect to the normal of the base area (21).

2. Medical device (10) according to Claim 1, wherein the **coupling means** (42) is arranged at an angle of between 50 degrees and 70 degrees with respect to the front area (23).

3. Medical device (10) according to one of the preceding claims, wherein a spatial region occupied in the housing (20) by the **light source** (70) is entirely arranged in the half which adjoins the base area (21) of the housing (20).

4. Medical device (10) according to one of the preceding claims, furthermore comprising:
an **attachment means** (51) on the **base area** (21) for attaching the housing (20) at least to a carrying case (90, 110) for the medical device (10) or a stand or another device.

5. Medical device (10) according to Claim 4, furthermore comprising:
a **carrying case** (90, 110), which is embodied to hold the medical device (10), wherein the attachment means (93) on the carrying case (90, 110) and the attachment means (51) on the base area (21) of the housing (20) of the medical device (10) are embodied for a detachable mechanical connection to one another.

6. Medical device (10) according to Claim 5, wherein the attachment means (51) at the base area (21) of the housing (20) comprises at least one groove or a web with a **dovetail** or trapezoidal cross section, and wherein the attachment means (93) on the carrying case (90) is complementary to the attachment means (51) on the base area (21) of the housing (20) and correspondingly comprises at least one web or groove with a **dovetail** or trapezoidal cross section.

7. Medical device (10) according to one of the preceding claims, wherein the rear side area (24) encloses a **recess** (60) in a U-shaped manner, in which recess an attachment means (64) is arranged for attaching a rechargeable battery to the medical device (10).

8. Medical device (10) according to Claim 7, wherein the attachment means (64) in the recess (60) is embodied for **alternative** or simultaneous attachment of a **rechargeable battery** or a cooling body (62) on the medical device (10) or of the medical device (10) on a **support** (96, 97).

9. Medical device (10) according to either of Claims 7 or 8, furthermore comprising:
a **cooling body** (62) in the recess (60), for dissipating waste heat from the light source (70), a power supply (75) integrated into the medical device (10) or another means (79).

10. Medical device (10) according to Claim 7, wherein
the attachment means (64) has a waist (65) into which a keyhole-shaped recess (102) in a housing wall (101) of a rechargeable battery can be mounted,
and, in particular, the cooling body (62) has a latching groove (68), which enables a latching connection to the rechargeable battery.

11. Medical device (10) according to one of the preceding claims, furthermore comprising:
a **cable holder** (54) on the rear side (14) of the housing (20), for keeping a cable for the medical device (10).

12. Medical device (10) according to one of the preceding claims, wherein the distance (s) of the **centre of gravity** (84) of the medical device (10) from the base area (21) is at most one third of the overall height (h) of the housing (20).

13. Medical device (10) according to one of the preceding claims, furthermore comprising:
a **holding means** (56, 57), which is partly integrated into the housing (20), for holding a vessel for a fluid to be used during an endoscopic examination.

## Revendications

1. Dispositif médical (10) pour soutenir un examen endoscopique, comprenant :
un boîtier (20) comprenant une face de fond (21), une face avant (23) et une face arrière (24) qui sont à chaque fois réalisées de manière essentiellement plane ;
une source de lumière (70) qui est disposée dans le boîtier (20) pour produire de la lumière pour éclairer un objet à examiner avec un endoscope ;
un dispositif d'accouplement (42) pour l'accouplement direct ou indirect d'une extrémité proximale d'un endoscope au dispositif médical (10) et pour transmettre de la lumière depuis le dispositif médical (10) jusqu'à l'endoscope ;
un écran (32) qui est disposé sur la face avant (23) du boîtier (20) pour observer une image prise au moyen de l'endoscope,
**caractérisé en ce que** la face avant (23) comprend l'écran (32) intégré dans celle-ci et la face avant (23) et la face arrière (24) sont inclinées vers l'arrière par rapport à la face de fond (21) à chaque fois suivant un angle compris entre 5 degrés et 15 degrés par rapport à la verticale à la face de fond (21).

2. Dispositif médical (10) selon la revendication 1, dans lequel le dispositif d'accouplement (42) est disposé suivant un angle compris entre 50 degrés et 70 degrés par rapport à la face avant (23).

3. Dispositif médical (10) selon l'une quelconque des revendications précédentes, dans lequel une région spatiale occupée par la source de lumière (70) dans le boîtier (20) est disposée exclusivement dans la moitié adjacente à la face de fond (21) du boîtier (20).

4. Dispositif médical (10) selon l'une quelconque des revendications précédentes, comprenant en outre :
un dispositif de fixation (51) au niveau de la face de fond (21) pour fixer le boîtier (20) au moins soit à une mallette de transport (90, 110) pour le dispositif médical (10) soit à un support ou à un autre dispositif.

5. Dispositif médical (10) selon la revendication 4, comprenant en outre :
une mallette de transport (90, 110) qui est réalisée pour recevoir le dispositif médical (10), le dispositif de fixation (93) au niveau de la mallette de transport (90, 110) et le dispositif de fixation (51) au niveau de la face de fond (21) du boîtier (20) du dispositif médical (10) étant réalisés de manière à pouvoir être connectés mécaniquement de manière amovible l'un à l'autre.

6. Dispositif médical (10) selon la revendication 5, dans lequel le dispositif de fixation (51) au niveau de la face de fond (21) du boîtier (20) comprend au moins une rainure ou une nervure de section transversale en forme de queue d'aronde ou trapézoïdale, et dans lequel le dispositif de fixation (93) au niveau de la mallette de transport (90) est complémentaire au dispositif de fixation (51) au niveau de la face de fond (21) du boîtier (20) et comprend de manière correspondante au moins une nervure ou une rainure de section transversale en forme de queue d'aronde ou trapézoïdale.

7. Dispositif médical (10) selon l'une quelconque des revendications précédentes, dans lequel la face arrière (24) entoure en forme de U un évidement (60) dans lequel est disposé un dispositif de fixation (64) pour la fixation d'un accumulateur au niveau du dispositif médical (10).

8. Dispositif médical (10) selon la revendication 7, dans lequel le dispositif de fixation (64) est réalisé dans l'évidement (60) en vue de la fixation alternative ou simultanée d'un accumulateur ou d'un corps de refroidissement (62) au niveau du dispositif médical (10), ou bien le dispositif médical (10) est réalisé au niveau d'un support (96, 97).

9. Dispositif médical (10) selon l'une quelconque des revendications 7 et 8, comprenant en outre :
un corps de refroidissement (62) dans l'évidement (60), pour évacuer la chaleur perdue de la source de lumière (70), d'une alimentation en puissance (75) intégrée dans le dispositif médical (10) ou d'un autre dispositif (79).

10. Dispositif médical (10) selon la revendication 7, dans lequel
le dispositif de fixation (64) présente une partie rétrécie (65) dans laquelle peut être accroché un évidement en forme de trou de serrure (102) dans une paroi de boîtier (101) d'un accumulateur,
et notamment le corps de refroidissement (62) présente une rainure d'encliquetage (68) qui permet une connexion par encliquetage à l'accumulateur.

11. Dispositif médical (10) selon l'une quelconque des revendications précédentes, comprenant en outre :
un dispositif de retenue de câble (54) au niveau du côté arrière (14) du boîtier (20), pour ranger un câble pour le dispositif médical (10).

12. Dispositif médical (10) selon l'une quelconque des revendications précédentes, dans lequel la distance (s) du centre de masse (84) du dispositif médical (10) depuis la face de fond (21) vaut au maximum un tiers de la hauteur totale (h) du boîtier (20).

13. Dispositif médical (10) selon l'une quelconque des revendications précédentes, comprenant en outre :
un dispositif de retenue (56, 57) qui est en partie intégré dans le boîtier (20), pour retenir un récipient pour un fluide à utiliser dans un examen endoscopique.
